# EUROPÄISCHE PATENTANMELDUNG

(11) **EP 0 786 467 A1**
(43) Veröffentlichungstag der Anmeldung: **30.07.1997**
(21) Anmeldenummer: 97100320.7
(22) Anmeldetag: 10.01.1997
(51) Int. Cl.: C07H 3/04, C07H 3/06, A61K 31/70

(54) **Mehrfach fucosylierte Dicarbonsäuren mit antiadhäsiven Eigenschaften**

(30) Priorität: 24.01.1996 DE 19602355
(71) Anmelder: HOECHST AKTIENGESELLSCHAFT, 65929 Frankfurt am Main (DE)
(72) Erfinder: Toepfer, Alexander, Dr., 65830 Kriftel (DE); Kretzschmar, Gerhard, Dr., 65760 Eschborn (DE); Bartnik, Eckart, Dr., 65205 Wiesbaden-Delkenheim (DE); Hüls, Christoph, Dr., 55263 Wackernheim (DE); Seiffge, Dirk, Dr., 55246 Mainz-Kostheim (DE)

(57) **Zusammenfassung**

Die Erfindung betrifft mehrfach fucosylierte Dicarbonsäurederivate mit antiadhäsiven Eigenschaften, ein Verfahren zu deren Herstellung, deren Verwendung sowie aus diesen hergestellte Arzneimittel und Diagnostika.

Diese mehrfach fucosylierten Dicarbonsäurederivate eignen sich zur Herstellung von Arzneimitteln oder Diagnostika für Krankheiten, welche mit einer übermäßigen, durch Selektinrezeptoren vermittelten Zelladhäsion in dem von der Krankheit betroffenen Gewebe einhergehen.

## Beschreibung

Die Erfindung betrifft mehrfach fucosylierte Dicarbonsäuren mit antiadhäsiven Eigenschaften, ein Verfahren zu deren Herstellung, deren Verwendung sowie aus diesen hergestellte Arzneimittel und Diagnostika.

Die Zirkulation von Blutzellen wie z.B. Leukozyten, Neutrophilen, Granulozyten und Monozyten ist auf molekularer Ebene ein vielstufiger, sehr komplexer Prozess, der nur in Teilschritten bekannt ist (Review: T.A.Springer, Cell 76, 301-314, 1994).

Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.

An diesem Vorgang sind zahlreiche spezifische Signalmoleküle wie z.B. Interleukine, Leukotriene und Tumornekrosefaktor (TNF), deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die drei Selektinrezeptoren bestimmen die Anfangsphase der Leukozytenadhäsion. E-Selektin wird auf Endothelzellen einige Stunden nach der Stimulierung beispielsweise durch Interleukin-1 (IL-1β) oder Tumornekrosefaktor (TNF-a) exprimiert, während P-Selektin in Blutplättchen und Endothelzellen gespeichert wird und nach Stimulierung durch Thrombin, Peroxidradikale oder Substanz P u.a. auf den Zelloberflächen präsentiert wird. L-Selektin wird dauerhaft auf Leukozyten exprimiert, wird im Verlauf der Entzündung jedoch rasch wieder von den Leukozyten abgespalten.

Die in der Anfangsphase entzündlicher Prozesse durch Selektin-Rezeptoren vermittelte Adhäsion von Leukozyten an Endothelzellen ist eine natürliche und notwendige Immunantwort auf diverse Entzündungsreize und Verletzungen des vasculären Gewebes.
Der Verlauf einer Reihe von akuten und chronischen Erkrankungen wird jedoch durch die übermäßige Adhäsion von Leukozyten und deren Infiltration in das betroffene Gerwebe sowie durch die Schädigung gesunden Gewebes im Sinne einer Autoimmunreaktion ungünstig beeinflußt. Dazu zählen beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie und By-Pass Operationen) auftretende Restenose.

Der natürliche Ligand mit der Struktur von SLeX wurde schon erfolgreich in Tierversuchen bei P-Selektin abhängigen Lungenverletzungen (M.S.Mulligan et.al., Nature 1993, 364, 149) und bei myokardialen Reperfusionsverletzungen (M.Buerke et.al., J.Clin.Invest. 1994, 93, 1140) verwendet. In ersten klinischen Prüfungen bei akuter Lungenentzündung soll die Verbindung in einer Dosis von 1-2 Gramm pro Tag und Patient eingesetzt werden (Mitteilung der Firma Cytel Corp,/ La Jolla (CA.) beim 2. Glycotechnology Meeting/CHI in La Jolla/USA am 16.-18. Mai 1994).

Diese hohe Wirkstoffdosis steht im Einklang mit der bekanntermaßen schwachen Affinität der natürlichen SLeX/A-Liganden zu den Selektin-Rezeptoren. So inhibiert SLeX in allen bekannten in vitro-Testsystemen die Zelladhäsion an Selektinrezeptoren erst bei einer relativ hohen Konzentration im Bereich von IC₅₀ ca. 1 mM (Jacob et.al., Biochemistry 1995, 34, 1210). In einigen Publikationen und Patentanmeldungen wurde inzwischen über Bemühungen berichtet, durch strukturelle Variation des Liganden zu fester bindenden Antagonisten zu gelangen. Ziel dieser Arbeiten ist die Bereitstellung wirksamerer Antagonisten, die auch potentiell in vivo bei geringerer Dosis einsetzbar wären.

Die Variation der für die Struktur-Wirkungs-Beziehung bislang als entscheidend angesehenen Fucose- und Neuraminsäurebausteine (B.K.Brandley et.al., Glycobiology 1993, 3, 633 und M.Yoshida et.al., Glycoconjugate J. 1993, 10, 3) erbrachte jedoch keine signifikant verbesserten Inhibitionswerte. Allein bei Variation des Glucosaminbausteins (Ersatz von GlcNAc durch Glucose und Azido- sowie Aminogruppen in der 2-Position von GlcNAc) ließ sich eine signifikant erhöhte Affinität an den E-Selektin-Rezeptor erreichen. Beim P-Selektin-Rezeptor wurde hingegen keine verbesserte Bindung erreicht.

Die IC₅₀-Daten dieser Oligosaccharid-Derivate soll für die Hemmung der Adhäsion von HL-60 und U-937 Zellen bei 0.12 mM (gegenüber 1.2-2.0 mM für SLeX) bei E-Selektin liegen. Von Nachteil ist hingegen, daß die Bindung an L-und P-Selektine mit > 5 mM stark beeinträchtigt wird (Dasgupta et.al., Posterpräsentation der Firma Glycomed Inc. anläßlich der Tagung in La Jolla in 5/94).
Generell blieben bisher alle Erfolge zur Verbesserung der Bindungsaffinität von SLeX-und SLeA-Derivaten auf den E-Selektinrezeptor beschränkt, denn mit dem P-Selektinrezeptor wurden nur schwache Inhibitionseffekte bei Inhibitorkonzentrationen von ca. 1 mM gefunden (R.M.Nelson et.al., J.Clin.Invest. 1993, 91, 1157).

Der Stand der Technik zur Bindungsaffinität modifizierter SLeX/A-Strukturen an Selektine wird in Pharmacochem. Libr. 1993, 20 (Trends in Drug Research), Seiten 33-40 referiert.

Die Aufgabe der vorliegenden Erfindung besteht in der Herstellung neuer Selektin-Liganden, welche eine im Vergleich zu den natürlichen Liganden deutlich stärkere Bindung zu den Rezeptoren aufweisen und sich außerdem leichter als diese synthetisieren lassen.

Die gestellte Aufgabe wird gelöst durch
1. eine Verbindung der Formel I 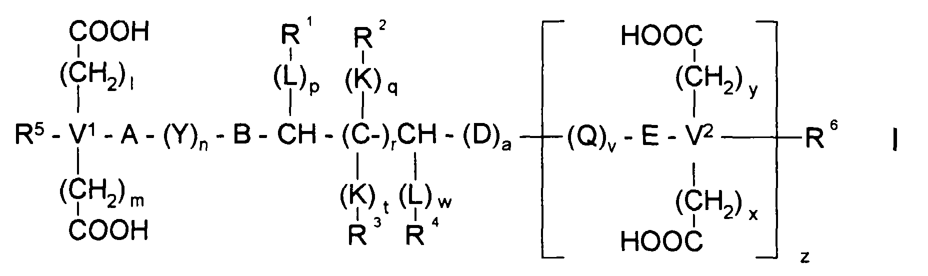 worin bedeuten
   - R⁵ und R⁶: unabhängig voneinander H, OH, COOH, NH₂, NHAc, -O(CH₂)_{c}X¹, (CH₂)_{c}X¹, CH₂O(CH₂)_{c}X¹ oder Z,
   - A,B,D,E und G: unabhängig voneinander O, S, -NH, -HN-C(O)-, -(O)C-NH-, -O-C(O)-, -(O)C-O-, NH-C(O)-O, O-C(O)-NH, NH-C(O)-NH, S-C(O)-, (S)C-O, O-C(S)-S, S-C(S)-O, NH-C(S)-S, S-C(S)-NH, -CH₂-, -O-CH₂-, CH₂-O-, CH₂-NH- oder NH-CH₂,
   - V¹ und V²: unabhängig voneinander ein Kohlenstoffatom oder ein Stickstoffatom, wobei für den zweiten Fall R⁵ bzw. R⁶ entfallen,
   - Q und Y: unabhängig voneinander -(CX²,X³)_{b}-, -(CX²,R⁷)_{b}-, -(CR⁷,R⁸)_{b}-, -CH₂-(CX²,X³)_{b}- oder einen gesättigten oder ungesättigten, fünf- oder sechsgliedrigen Carbo- oder Heterocyclus oder eine Kombination aus der Kette -(CX²,X³)_{b}-, -(CX²,R⁷)_{b}-, -(CR⁷,R⁸)_{b}- und einem Carbo oder Heterocyclus, wobei
   - R⁷ und R⁸: unabhängig voneinander H, OH, COOH, NH₂, NH-C(O)-CH₃, O(CH₂)_{d}X¹ oder CH₂O(CH₂)_{d}X¹ und
   - X¹, X² und X³: unabhängig voneinander H, NH₂, COOH, OH, CH₂OH, CH₂NH₂, C₁-C₂₀-Alkyl oder C₆-C₁₀-Aryl bedeuten,
   - K und L: unabhängig voneinander H-C-GZ oder CH₂,
   - R¹,R²,R³ und R⁴: unabhängig voneinander H, OH oder G-Z,
   - Z: ein Pyranosid, ein über die C6-Position verknüpfter Pyranosylrest, ein über die C6-Position verknüpftes Alkyl-Pyranosid,ein Furanosid, ein über die C5-Position verknüpftes Alkyl-Furanosid oder ein Polyalkohol, bei welchem eine oder mehrere Hydroxylgruppen unabhängig voneinander durch R⁷ oder R⁸ substituiert sein können, welcher über eine beliebige Position mit G verknüpft ist,
   und die Indizes
   - a,b,c,d,l,m,n,p,q,t,v,w,x und y: unabhängig voneinander eine ganze Zahl von 0 bis 20, sowie
   - r und z: unabhängig voneinander 0 oder 1, wobei für den Fall, daß r die Zahl 0 bedeutet, q und t ebenfalls 0 bedeuten und R² und R³ entfallen,
2. vorzugsweise durch eine Verbindung der Formel I, welche dadurch gekennzeichnet ist, daß
   - R⁵: H,
   - V¹: C,
   - A und Y: CH₂,
   - B: O-CH₂,
   - D: CH₂-O,
   - r,p und w: die Zahl 0 und
   - R¹ und R⁴: G-Z bedeuten,
3. vorzugsweise dadurch gekennzeichnet, daß
   - Z: 1,
   - Q und E: CH₂,
   - V²: C und
   - R⁶: H bedeuten,
4. besonders bevorzugt dadurch gekennzeichnet, daß
   - l,m,y und x: die Zahl 0,
   - G: O und
   - Z: ein Pyranosid bedeuten,
5. wobei das Pyranosid Z vorzugsweise ein L-Fucosid ist, beispielsweise 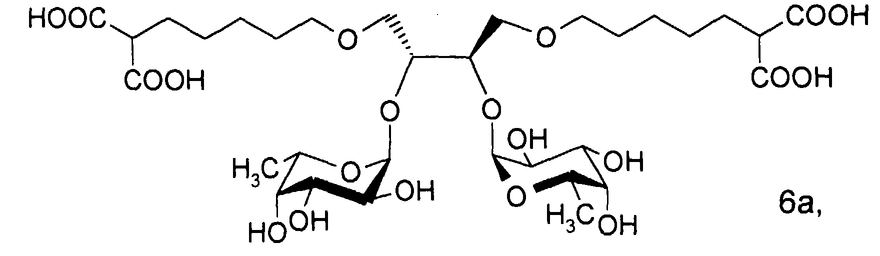
6. Vorzugsweise ist die Verbindung der Formel I mit den unter Nr. 2 dargestellten Merkmalen, ferner dadurch gekennzeichnet, daß
   - z: die Zahl 0 und
   - R⁶: Z bedeuten,
7. vorzugsweise dadurch gekennzeichnet, daß
   - l und m: die Zahl 0,
   - G: O und
   - Z: ein Pyranosid bedeuten,
8. wobei das Pyranosid Z besonders bevorzugt ein L-Fucosid ist, beispielsweise 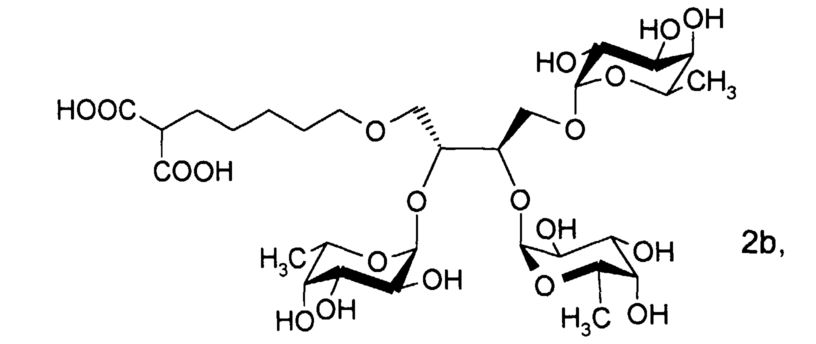
9. Eine weitere bevorzugte Ausgestaltung der vorliegenden Erfindung ist eine Verbindung der Formel I, die sich dadurch auszeichnet, daß
   - a: 1,
   - R¹,R⁴ und R⁵: H,
   - V¹: C,
   - A und Y: CH₂,
   - B und D: O,
   - p und w: die Zahl 0,
   - r: 1,
   - K: CH₂ und
   - R² und R³: GZ bedeuten,
10. vorzugsweise dadurch gekennzeichnet, daß
   - z: 1,
   - Q und E: CH₂,
   - V²: C und
   - R⁶: H bedeuten,
11. besonders bevorzugt dadurch gekennzeichnet, daß
   - l,m,x und y: die Zahl 0,
   - q und t: 1,
   - G: O und
   - Z: ein Pyranosid bedeuten, wobei das Pyranosid Z vorzugsweise ein L-Fucosid ist, beispielsweise 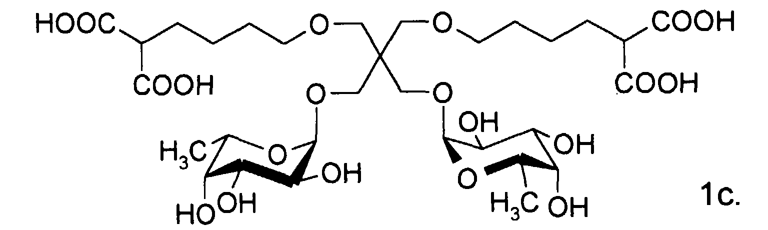
13. Eine weitere Ausgestaltung der vorliegenden Erfindung ist eine Verbindung der Formel I, welche sich dadurch auszeichnet, daß
   - R⁵: H,
   - V¹: C,
   - A und Y: CH₂,
   - B und D: O und
   - r: die Zahl 0 bedeuten,
14. vorzugsweise dadurch gekennzeichnet, daß
   - z: 1,
   - R⁶: H,
   - Q und E: CH₂ und
   - V²: C, bedeuten,
15. besonders bevorzugt dadurch gekennzeichnet, daß
   - l,m,y und x: die Zahl 0,
   - p und w: 2,
   - L: H-C-GZ,
   - R¹ und R⁴: H,
   - G: O und
   - Z: ein Pyranosid bedeuten, wobei
16. das Pyranosid Z besonders bevorzugt ein L-Fucosid ist, beispielsweise 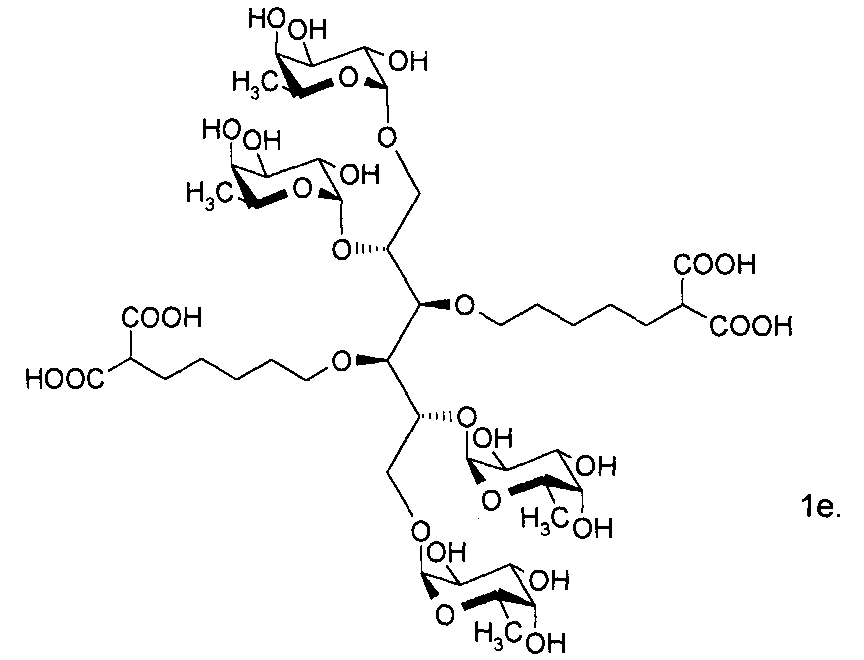
17. Eine weitere Ausgestaltung der vorliegenden Erfindung ist eine Verbindung der Formel I, welche dadurch gekennzeichnet ist, daß
   - a,p,r,w und z: die Zahl 0,
   - R⁵ und R⁶: H,
   - V¹: C,
   - A und Y: CH₂,
   - B: O-CH₂ und
   - R¹ und R⁴: G-Z bedeuten,
18. bevorzugt dadurch gekennzeichnet, daß
   - l und m: die Zahl 0,
   - G: O und
   - Z: ein Pyranosid bedeuten, wobei
19. das Pyranosid vorzugsweise ein L-Fucosid ist, beispielsweise 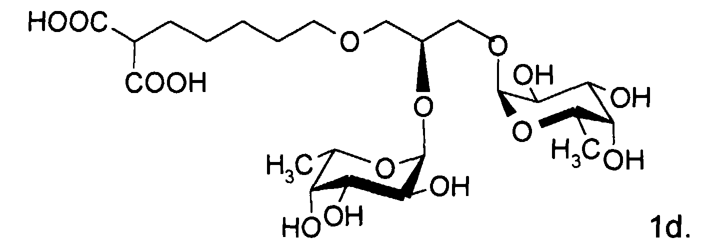
20. Die eingangs gestellte Aufgabe wird ferner gelöst durch ein Verfahren zur Herstellung einer Verbindung der Formel I, welches sich dadurch auszeichnet, daß man zunächst unter Alkylierung, Acylierung oder Glycosylierung einer funktionellen Gruppe L¹ oder zweier funktioneller Gruppen L¹ und L², gleichzeitig oder nacheinander, eines Akzeptors der Formel II 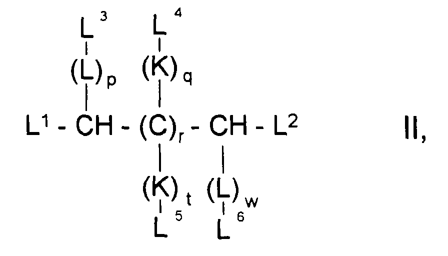 bei welchem die übrigen funktionellen Gruppen sowie die funktionellen Gruppen Lⁿ (n = 2-6 bzw. 3-6) notwendigenfalls geschützt vorliegen, mittels eines mit einer aktivierten funktionellen Gruppe L⁷ versehenen Donors III,

   L⁸ - A - (Y)ₙ - B - L⁷ III

   und für den Fall, daß auch L² ungeschützt vorliegt, mittels einer mit einer aktivierten funktionellen Gruppe L⁷ versehenen Donors IV

   L⁷ - (D)ₐ - (Q)ᵣ - E - L⁸ IV,

   deren übrige funktionelle Gruppe und die funktionelle Gruppe L⁸ notwendigenfalls Schutzgruppen tragen, Zwischenverbindung V 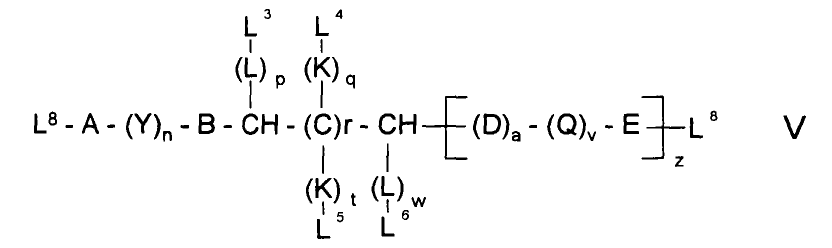 herstellt, wobei die Donoren III und IV verschieden oder identisch sein können, wonach die funktionellen Gruppen der Zwischenverbindung V an den Atomgruppen K und L und gegebenenfalls die Schutzgruppen der funktionellen Gruppen L³ bis L⁶ und L⁸ selektiv entfernt werden und die selektiv entschützte Zwischenverbindung V mit einem oder mehreren Glycosyl- oder Polyoldonoren VI

   L⁹ - Z VI

   umgesetzt wird, wobei L⁹ eine aktivierte funktionelle Gruppe darstellt und die übrigen funktionellen Gruppen des Donors VI notwendigenfalls geschützt sind, und nach selektiver Entschützung der funktionellen Gruppen L⁸ mit dem Alkyldonor VII 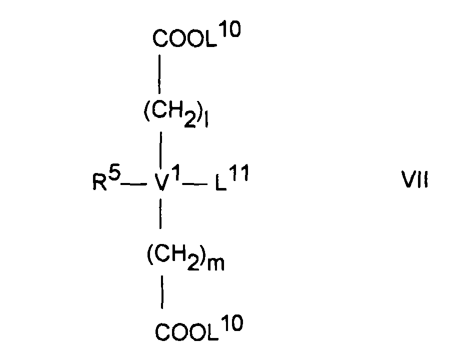 und gegebenenfalls mit dem
   Alkyldonor VIII 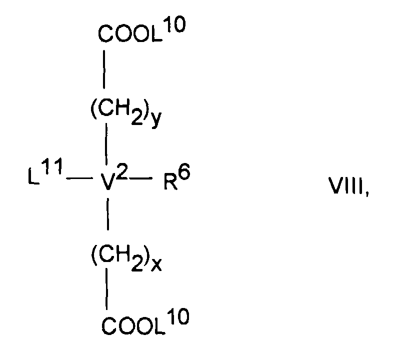 wobei L¹⁰ eine Carboxylschutzgruppe und L¹¹, gegebenenfalls in Verbindung mit V¹ bzw. V², eine alkylierende Gruppe darstellt, alkyliert und schließlich durch Entfernung sämtlicher Schutzgruppen in eine Verbindung der Formel I gemäß einem der Ansprüche 1 bis 16 überführt wird, wobei sämtliche übrigen Variablen die unter Nr. 1 genannten Bedeutungen haben.
21. Wahlweise kann der Akzeptor II auch zuerst mit einem oder mehreren Glycosyl- oder Polyoldonoren VI und anschließend, mit Donor IV zur Zwischenverbindung V umgesetzt werden.

a) Die unter Nr. 5 und 8 beschriebenen Verbindungen lassen sich unter Verwendung des erfindungsgemäßen Verfahrens vorzugsweise dadurch herstellen, daß man als Akzeptor II (-)-2,3-O-Isopropyliden-L-threitol, als Donoren III und IV 5-Allyloxy-1-p-toluolsulfonyloxypentan und als Glycosyldonor VI O-(2,3,4-Tri-O-benzyl-a/β-L-fucopyranosyl)-trichloracetimidat verwendet. Nach Abspaltung der beiden Allylgruppen und Tosylierung oder Bromierung der enstehenden Hydroxylgruppen wird mit Malonsäuredimethylester, Alkyldonor VII, alkyliert und anschließend entschützt.
b) Die unter Nr. 11 beschriebene Verbindung läßt sich unter Verwendung des erfindungsgemäßen Verfahrens vorzugsweise dadurch herstellen, daß man als Akzeptor II Isopropyliden-pentaerythrit verwendet und wie unter a) beschrieben weiter umsetzt.
c) Die unter Nr. 16 beschriebene Verbindung läßt sich unter Verwendung des erfindungsgemäßen Verfahrens vorzugsweise dadurch herstellen, daß man als Akzeptor II 1,2:5,6-di-O-isopropyliden-D-mannit verwendet und wie unter a) beschrieben weiter umsetzt.
d) Die unter Nr. 19 beschriebene Verbindung läßt sich unter Verwendung des erfindungsgemäßen Verfahrens vorteilhaft dadurch herstellen, daß man als Akzeptor II D-α/β-Isopropylidenglycerol verwendet und wie unter a) beschrieben weiter mit dem Alkyldonor VII umsetzt.

Die erfindungsgemäßen Verbindungen der Formel I können trotz ihrer geringeren Molmasse als Sialyl Lewis X eine höhere Affinität zu den natürlichen Rezeptoren, beispielsweise zu E- und P- Selektin, als dieses haben. Dies kann anhand der nachfolgend beschriebenen Zelladhäsionsassays nachgewiesen werden.

Primärassays zur Untersuchung der Wirkung der Verbindungen gemäß der vorliegenden Erfindung auf die Zellanheftung an rekombinante, lösliche Selektin-Fusionsproteine.

Um die Wirksamkeit der erfindungsgemäßen Verbindungen auf die Interaktion zwischen den L- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von L- bzw. P-Selektin und der konstanten Region eines humanen Immunglobulins der Subklasse IgG1 verwendet.

### Herstellung von L-Selektin-IgG1

Zur Herstellung von löslichem L-Selektion-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F. M., Brent, R., Kingston, R. E., Moore, D. D., Seidman, J. G., Struhl, K. und Smith, J. A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben. (Walz, G., Aruffo, A., Kolanus, W., Bevilacqua, M. und Seed, B. 1990. Recognition by ELAM-1 of the sialyl-Lex determinant on myeloid and tumor cells. Science 250 1132-1135.)

### Herstellung von P-Selektin-IgG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der oben dargestellten Herstellung von L-Selektin-IgG1.

Aruffo, A., Kolanus, W., Walz, G., Fredman, P. und Seed, B. 1991. CD62/-P-Selectin recognition of myeloid and tumor cell sulfatides. Cell 67, 35-44.

### Herstellung von CD4-IgG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1. (Zettelmeissl, G., Gregersen, J.-P., Duport, J. M., Mehdi, S., Reiner, G. und Seed, B. 1990. Expression and characterization of human CD4: Immunoglobulin Fusion Proteins. DNA and Cell Biology 9, 347-353.)

### Durchführung des HL60 Zelladhäsionsassays auf rekombinanten, löslichen Adhäsionsmolekülen

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der
   Bindungspuffer hat die Zusammensetzung: 50 mM Hepes, pH 7,5;
   100 mM NaCl; 1 mg/ml BSA;
   2 mM MgCl₂; 1 mM CaCl₂; 3 mM MnCl₂; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl₂; 1 mM CaCl₂; 3 mM MnCl₂; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

Leukozyten-Adhäsion - Prüfung der Wirksamkeit der erfindungsgemäßen Verbindungen in vivo (Intravitalmikroskopie an der Ratte):

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune system. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301-314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphnuclear leukocytes to blood vessel walls. J. Physiol. 222, 447-474, 1972). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspectives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation der Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (Angabe der Ergebnisse in Prozenten).

### Ergebnisse:

- Verbindung 6a:: Dosis: 10 mg/kg; Applikation: i.v.; Spezies: SPRD(m); Gewicht: 298 ± 17,72 g; Anzahl der Gefäße: 14; Gefäßdurchmesser: 26 ± 2 µm; Leukozyten: (6,0 ± 1,09)·10³/mm³; Fibrinogen 130 ± 6,95 mg/100 ml; Inhibierung: 39 %.

Reperfusionsmodell zur Untersuchug des Einflusses der Adhäsion von Neutrophilen im Verlauf der Ischämie/Reperfusion am offenen Kaninchen-Herzen.

Die Herzen werden bei konstantem Druck nach der Langendorff-Technik mit Nährlösung sowie mit/ohne Leukozyten bzw. Wirkstoff perfundiert. Danach wird eine Ischämie durch Unterbinden der linken Koronarartherie (30 min) hervorgerufen. Nach Reperfusion (30 min) wird die Leukozytenakkumulierung histologisch ausgewertet. Im Versuchsverlauf werden außerdem an 256 Elektroden Potentiale und Arrhythmien gemessen (Gesamtversuchsdauer ca. 90 min). Bei 6 von 7 unbehandelten mit Leukozyten perfundierten Herzen treten ausgeprägte Arrhythmien aufgrund der Leukozyteninfiltration auf, während die mit einem Wirkstoff (RGDS-Peptide, Chondroitinsulfat) behandelten Herzen verminderte Leukozyten Akkumulierung und Arrhythmien entwickeln. Die Verbindungen gemäß der vorliegenden Erfindung sowie deren physiologisch verträglichen Salze eignen sich aufgrund ihrer wertvollen pharmakologischen Eigenschaften sehr gut zur Anwendung als Heilmittel bei Säugern, insbesondere dem Menschen.

Die vorliegende Erfindung betrifft daher ferner ein Arzneimittel enthaltend wenigstens eine Verbindung der Formel I oder deren pharmakologisch verträgliche Salze sowie deren Verwendung für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen, beispielsweise Autoimmunerkrankungen wie Rheuma oder Herz-Kreislauf-Erkrankungen, wie Reperfusionsverletzungen, Ischämie oder Herzinfarkt.

Als pharmakologisch verträgliche Salze der Verbindung der Formel I sind Salze mit anorganischen und organischen Basen, beispielsweise NaOH, KOH, Mg(OH)₂, Ca(OH)₂, Diethanolamin, Ethylendiamin, oder mit Aminosäuren, wie Arginin, Lysin oder Glutaminsäure, besonders geeignet. Sie werden nach Standardvorschriften hergestellt.

Die Arzneimittel eignen sich im besonderen zur Behandlung von akuten und chronischen Entzündungen, die sich pathophysiologisch durch eine Störung der Zellzirkulation, beispielsweise von Lymphozyten, Monozyten und neutrophilen Granulozyten, kennzeichnen lassen. Hierzu zählen Autoimmunerkrankungen wie akute Polyarthritis, rheumatoide Arthritis und Insulin-abhängige Diabetes (Diabetes mellitus IDDM) akute und chronische Transplantatabstoßungen, Schocklunge (ARDS, adult respiratory distress syndrome), entzündliche und allergische Hauterkrankungen wie beispielsweise Psoriasis und Kontakt-Ekzeme, Herz-Kreislauf-Erkrankungen wie Myokardinfarkt, Reperfusionsverletzungen nach Thrombolyse , Angioplastie oder By-Pass-Operationen, septischer Schock und systemischer Schock. Eine weitere potentielle Indikation ist die Behandlung metastasierender Tumoren, denn Tumorzellen tragen Oberflächenantigene, die sowohl Sialyl-Lewis-X als auch Sialyl-Lewis-A-Strukturen als Erkennungsepitope besitzen. Darüberhinaus können diese Arzneimittel, die stabil im sauren Milieu des Magens sind, zur antiadhäsiven Therapie von Helicobacter Pylori und verwandten Mikroorganismen ggf. auch in Kombination mit Antibiotika eingesetzt werden. Ferner ist mit Hilfe dieser Arzneimittel eine Therapie der cerebralen Form der Malaria denkbar.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche exprimiert werden und demzufolge die zu verabreichende Tagesdosis geringer ist als bei stark erkrankten Patienten.

Die erfindungsgemäßen Arzneimittel werden dadurch hergestellt, daß man eine Verbindunmg gemäß der vorliegenden Erfindung mit üblichen Träger- sowie gegebenenfalls Zusatz- und/oder Hilfsstoffen in die bzw. eine geeignete Darreichungsform bringt.

Ferner ist die Verwendung von Verbindungen gemäß der Formel I für die Herstellung eines Mittels zur Diagnose einer Krankheit, welche mit einer übermäßigen, durch Selektinrezeptoren vermittelten Zelladhäsion in dem von der Krankheit betroffenen Gewebe einhergeht, denkbar.

Beispiele für die Herstellung der erfindungsgemäßen Verbindungen:

### Beispiel 1

a) Synthese von 1,4-Bis(5-allyloxy-1-pentyloxy)-D-threitol (1a) und 1-(5-allyloxy-1-pentyloxy)-D-threitol (1b):
   Eine Mischung aus 2,3-O-Isopropyliden-D-threitol (1,38 g, 8,51 mmol) und Natriumhydrid (572 mg, 23,83 mg) in Dimethylformamid (100 ml) wird 30 min bei Raumtemperatur gerührt. Dann wird bei 0 °C
   5-Allyloxy-1-pentyloxy-toluolsufonat (6,6 g, 22,19 mmol) zugetropft.
   Anschließend läßt man 2 Tage bei Raumtemperatur rühren. Zur Aufarbeitung werden Wasser (20 ml) und Diethylether (400 ml) zugegeben. Die organische Phase wird mit Wasser (3 x 140 ml) gewaschen, über Natriumsulfat getrocknet, filtriert und eingeengt. Der Rückstand wird 45 min bei 60 °C mit 50%iger Trifluoressigsäure (250 ml) behandelt. Nach dem Einengen wird der Rückstand chromatografisch Toluol/Aceton 5:1 → 4:1 → 3:1 → 2:1 → 1:1 gereinigt. Man erhält 1a (2,1 g, 67%) und 1b (284 mg, 13%).
   Werden statt 2,8 Äquivalenten 5-Allyloxy-1-pentyloxy-toluolsufonat nur 0.8 Äquivalente verwendet, so läßt sich die Ausbeute von 1b auf über 60% steigern.
   1a: ¹H-NMR (300 MHz, CDCl₃): δ = 1.42 (m, 4H, 2 CH₂), 1.60 (m, 8H, 4 (CH₂), 2.86 (bd, 2H, 2 OH), 3.42 (m, 12H, 6 CH₂), 3.82 (m, 2H, 2-H_{threit.}, 3-H_{threit.}), 3.96 (m, 4H, 2 CH2-CH =CH2), 5.22 (m, 4H, 2 CH2-CH =CH2), 5.92 (m, 2H, 2 CH2-CH=CH2).
   1b: ¹H-NMR (300 MHz, CDCl₃): δ = 1.42 (m, 2H, CH₂), 1.60 (m, 4 H, 2 CH₂), 3.42-3.84 (m, 13H, 4 CH₂, 2-H_{threit.}, 3-H_{threit.}, 3 OH), 3.96 (m, 2H, CH2-CH =CH2), 5.22 (m, 2H, CH2-CH =CH2), 5.92 (m, 1H, CH2-CH=CH2).
b) Synthese von 1,4-Bis(5-allyloxy-1-pentyloxy)-2,3-bis(2,3,4-tri-O-benzyl-α-L-fucopyranosyl)-D-threitol (2a):
   Zu einer Lösung aus 1a (1,15 g, 3,09 mmol) in Diethylether (30 ml) gibt man eine 0,1 M Trimethylsilyltrifluormethansulfonat-Lösung (0,9 ml). Sofort danach wird eine Lösung von O-(2,3,4-Tri-O-benzyl-a/β-L-fucopyranosyl)trichloracetimidat (5,0 g, 8,6 mmol) in Diethylether (10 ml) binnen 5 min tropfenweise zugegeben. Nach 30 min wird Natriumhydrogencarbonat (0,25 g) zugegeben, filtriert, eingengt und der Rückstand chromatografiert (Hexan/Essigester 4:1 → 3:1). Man erhält 2a (3,21 g, 86%).
   ¹H-NMR (300 MHz, CDCl₃): δ = 1,10 (d, 3 H, CH_{3fuc}), 5.18 (m, 4H, 2 CH2-CH =CH2), 5.88 (m, 2H, 2 CH2-CH=CH2).
c) Synthese von 1,4-Bis(5-hydroxy-1-pentyloxy)-2,3-bis(2,3,4-tri-O-benzyl-α-L-fucopyranosyl)-D-threitol (3a):
   Eine Mischung aus 2a (3,21 g, 2,66 mmol) und Rhodium-tris-(triphenylphosphin)-chlorid (0,247 mmol, 0,267 mmol) in Ethanol/Wasser (9:1, 100 ml) wird 2 h unter Rückfluß gekocht. Nach dem Einengen wird der Rückstand mittels Chromatografie (Toluol/Aceton/Methanol 5:1:0,1) gereinigt. Man erhält 3a (1,53 g, 51%).
d) Synthese von 1,4-Bis(5-brom-1-pentyloxy)-2,3-bis(2,3,4-tri-O-benzyl-α-L-fucopyranosyl)-D-threitol (4a):
   Zu einer Lösung aus 3a (7,7 g, 6,86 mmol) und Triphenylphosphin (3,6 g, 13,72 mmol) in Dichlormethan (250 ml) gibt man bei 0 °C Triethylamin (3,8 ml, 27,44 mmol) und 1,2-Dibromtetrachlorethan (4,47 g, 13,72 mmol) hinzu. Nach 90 min wird eingeengt und der Rückstand chromatografisch Hexan/Essigester (3:1) gereinigt. Ausbeute an 4a: 5,8 g (68%).
e) Synthese von 1,4-Bis(5-malonyl-1-pentyloxy)-2,3-bis(2,3,4-tri-O-benzyl-α-L-fucopyranosyl)-D-threitol (5a):
   Eine Mischung aus 4a (5,8 g, 4,64 mmol), Malonsäuredimethylester (243 ml), Kaliumcarbonat (12,49 g) und Dibenzo-18-crown-6 (3,35 g) wird 3 h bei 100 °C gerührt. Zur Aufarbeitung wird, nach dem Abkühlen, mit Dichlormethan (2,5 l) verdünnt, über Kieselgur abgesaugt, und die organische Phase abwechselnd mit Wasser (110 ml) und Trockeneis behandelt, bis der pH-Wert der wässrigen Phase neutral ist. Es wird über Natriumsulfat getrocknet, filtriert, eingengt und der Rückstand chromatografiert (Hexan/Essigester 4:1 → 3:1 → 2:1). Man erhält 5a (5,6 g, 94%).
   ¹H-NMR (300 MHz, CDCl₃): δ = 1,10 (δ, 6H, 2 CH_{3fuc}), 1,26 (m, 12H, 6 CH₂), 1,47 (m, 4H), 1,83 (m, 4H), 3,70 (2 s, 12H, 4 COOCH₃), 5,29 (d, 2H, 2 1-H_{fuc}).
f) Synthese von 1,4-Bis(5-malonyl-1-pentyloxy)-2,3-bis(α-L-fucopyranosyl)-D-threitol (6a):
   Eine Mischung aus 5a (2,8 g, 2,19 mmol) und Palladiumkohle (10%, 2,8 g) in Methanol/Dioxan (10:1, 330 ml) wird 24 h unter Normaldruck in einer Wasserstoffatmosphäre hydriert. Die Palladiumkohle wird abfiltriert, der Rückstand eingeengt und mit 1 M Natronlauge (100 ml) behandelt. Nach 2 h wird mit Amberlite IR-120 neutralisiert und über RP-Kieselgel (C₁₈ Bakerbond 60 Å) mit Wasser/Methanol 9:1 → 1:9 gereinigt. Man erhält 6a (1,11 g, 67%).
   ¹H-NMR (300 MHz, D₂O): δ = 1,17 (d, 6H, 2 CH_{3fuc}), 1,26 (m, 4H, 2 CH₂), 1,52 (m, 4H, 2 CH₂), 1,66 (m, 4H, 2 CH₂) 4,20 (q, 2H, 2 5-H_{fuc}), 5,07 (d, 2H, 2 1-H_{fuc}).

### Beispiel 2

a) Synthese von 1-(5-malonyl-1-pentyloxy)-2,3,4-tris(α-L-fucopyranosyl)-D-threitol (2b):
   Verbindung 2b wird analog zu 6a synthetisiert, mit dem Unterschied, daß statt 1a, 1b verwendet wird.
   ¹H-NMR (300 MHz, D₂O): δ = 1,18 (3 d, 9H, 3 CH_{3fuc}), 1,26 (m, 2H, CH₂), 1,53 (m, 2H, CH₂), 1,67 (m, 2H, CH₂), 4,84, 5,06, 5,16 (3 d, 3H, 3 1-H_{fuc}).

### Beispiel 3

a) Synthese von Bis(4-malonyl-1-butyloxy)-bis(α/β-L-fucopyranosyl)pentaerythrit (1c):
   Verbindung 1c wird analog zu 6a synthetisiert, mit dem Unterschied, daß statt 2,3-O-Isopropyliden-D-threitol, O-Isopropyliden-pentaerythrit verwendet wird.
   ¹H-NMR (300 MHz, D₂O): δ = 1,10, 1,12 (2 d, 6H, 2 CH_{3fuc}), 4,17, 4,19, 4,70 (3 d, 2H, 2 1-H_{fuc}).

### Beispiel 4

a) Synthese von 1,2-bis(α-L-fucopyranosyl)-3-(5-malonyl-1- pentyloxy)-D-glycerol (1d):
   Verbindung 1d wird analog zu 6a synthetisiert, mit dem Unterschied, daß statt 2,3-O-Isopropyliden-D-threitol, D-α/β-Isopropylidenglycerol verwendet wird.
   ¹H-NMR (300 MHz, D₂O): δ = 1,10, (2d, 6H, 2 CH_{3fuc}), 4,75, 4,96 (2 d, 2H, 2 1-H_{fuc}).

### Beispiel 5

a) Synthese von 1,2,5,6-Tetrakis (α-L-fucopyranosyl)-3,4-bis(5-malonyl-1-pentyloxy)- D-mannit (1e):
   Verbindung 1e wird analog zu 6a synthetisiert, mit dem Unterschied, daß statt 2,3-O-Isopropyliden-D-threitol, 1,2:5,6-Di-O-isopropyliden-D-mannit verwendet wird.
   ¹H-NMR (300 MHz, D₂O): δ = 1,10, (d, 12H, 4 CH_{3fuc}), 4,26, 4,79, 5,07 (3 d, 2H, 2 1-H_{fuc}).

## Patentansprüche

1. Verbindung der Formel I 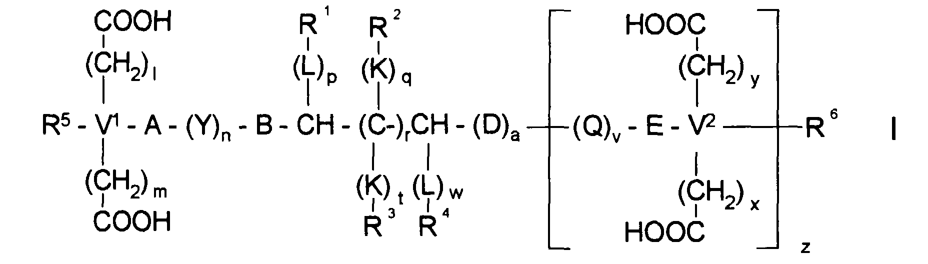 worin bedeuten
R⁵ und R⁶ unabhängig voneinander H, OH, COOH, NH₂, NHAc, O(CH₂)_{c}X¹ , (CH₂)_{c}X¹, CH₂O(CH₂)_{c}X¹ oder Z,
A,B,D,E und G unabhängig voneinander O, S, -NH, -HN-C(O)-, -(O)C-NH-, -O-C(O)-, -(O)C-O-, NH-C(O)-O, O-C(O)-NH, NH-C(O)-NH, S-C(O)-, (S)C-O, O-C(S)-S, S-C(S)-O, NH-CS-(S), S-C(S)-NH, CH₂, -O-CH₂, CH₂-O-, CH₂-NH- oder NH-CH₂,
V¹ und V² unabhängig voneinander ein Kohlenstoffatom oder ein Stickstoffatom, wobei für den zweiten Fall R⁵ bzw. R⁶ entfallen,
Q und Y unabhängig voneinander -(CX²,X³)_{b}-, -(CX²,R⁷)_{b}-, -(CR⁷,R⁸)_{b}-, -CH₂-(CX²,X³)_{b}- oder einen gesättigten oder ungesättigten, fünf -oder sechsgliedrigen Carbo-oder Heterocyclus oder eine Kombination aus der Kette -(CX²,X³)_{b}-, -(CX²,R⁷)_{b}-, -(CR⁷,R⁸)_{b}- und einem Carbo oder Heterocyclus, wobei
R⁷ und R⁸ unabhängig voneinander H, OH, COOH, NH₂, NH-C(O)-CH₃, O(CH₂)_{d}X¹ oder CH₂O(CH₂)_{d}X¹ und
X¹,X² und X³ unabhängig voneinander H, NH₂, COOH, OH, CH₂OH, CH₂NH₂, C₁-C₂₀-Alkyl oder C₆-C₁₀-Aryl bedeuten,
K und L unabhängig voneinander H-C-GZ oder CH₂,
R¹,R²,R³ und R⁴ unabhängig voneinander H, OH oder G-Z,
Z ein Pyranosid, ein über die C6-Position verknüpfter Pyranosylrest, ein über die C6-Position verknüpftes Alkyl-Pyranosid,ein Furanosid, ein über die C5-Position verknüpftes Alkyl-Furanosid oder ein Polyalkohol, bei welchem eine oder mehrere Hydroxylgruppen unabhängig voneinander durch R⁷ oder R⁸ substituiert sein können, welcher über eine beliebige Position mit G verknüpft ist,
und die Indizes
a,b,c,d,l,m,n,p,q,t,v,w,x und y unabhängig voneinander eine ganze Zahl von 0 bis 20, sowie
r und z unabhängig voneinander 0 oder 1, wobei für den Fall, daß r die Zahl 0 bedeutet, q und t ebenfalls 0 bedeuten und R² und R³ entfallen.

2. Verbindung nach Anspruch 1, dadurch gekennzeichnet, daß
a 1,
R⁵ H,
V¹ C,
A und Y CH₂,
B O-CH₂,
D CH₂-O,
r,p und w die Zahl 0 und
R¹ und R⁴ G-Z bedeuten.

3. Verbindung nach Anspruch 1 oder 2, dadurch gekennzeichnet, daß
Z 1,
Q und E CH₂,
V² C und
R⁶ H bedeuten.

4. Verbindung nach einem der Ansprüche 1 bis 3, dadurch gekennzeichnet, daß
l,m,y und x die Zahl 0,
G O und
Z ein Pyranosid bedeuten.

5. Verbindung nach einem der Ansprüche 1 bis 4, dadurch gekennzeichnet, daß Z ein L-Fucosid ist.

6. Verbindung nach Anspruch 1 oder 2, dadurch gekennzeichnet, daß
z die Zahl 0 und
R⁶ Z bedeuten.

7. Verbindung nach einem der Ansprüche 1, 2 oder 6, dadurch gekennzeichnet, daß
l und m die Zahl 0,
G O und
Z ein Pyranosid bedeuten.

8. Verbindung nach einem der Ansprüche 1, 2, 6 oder 7, dadurch gekennzeichnet, daß Z ein L-Fucosid ist.

9. Verbindung nach Anspruch 1, dadurch auszeichnet, daß
a 1,
R¹,R⁴ und R⁵ H,
V¹ C,
A und Y CH₂,
B und D O,
p und w die Zahl 0,
r 1,
K CH₂ und
R² und R³ GZ bedeuten.

10. Verbindung nach Anspruch 1 oder 9, dadurch gekennzeichnet, daß
z 1,
Q und E CH₂,
V² C und
R⁶ H bedeuten.

11. Verbindung nach einem der Ansprüche 1, 9 oder 10, dadurch gekennzeichnet, daß
l,m,x und y die Zahl 0,
q und t 1,
G O und
Z ein Pyranosid bedeuten.

12. Verbindung nach einem der Ansprüche 1 oder 9 bis 11, dadurch gekennzeichnet, daß Z ein L-Fucosid ist.

13. Verbindung nach Anspruch 1, dadurch auszeichnet, daß
a 1,
R⁵ H,
V¹ C,
A und Y CH₂,
B und D O und
r die Zahl 0 bedeuten.

14. Verbindung nach Anspruch 1 oder 13, dadurch gekennzeichnet, daß
z 1,
R⁶ H,
Q und E CH₂ und
V² C bedeuten.

15. Verbindung nach einem der Ansprüche 1, 13 oder 14, dadurch gekennzeichnet, daß
l,m,y und x die Zahl 0,
p und w 2,
L H-C-GZ,
R¹ und R⁴ H,
G O und
Z ein Pyranosid bedeuten.

16. Verbindung nach einem der Ansprüche 13 bis 15, dadurch gekennzeichnet, daß Z ein L-Fucosid ist.

17. Verbindung nach Anspruch 1, dadurch gekennzeichnet ist, daß
a,p,r,w und z die Zahl 0,
R⁵ und R⁶ H,
V1 C,
A und Y CH₂,
B O-CH₂ und
R¹ und R⁴ G-Z bedeuten.

18. Verbindung nach Anspruch 1 oder 17, dadurch gekennzeichnet, daß
l und m die Zahl 0,
G O und
Z ein Pyranosid bedeuten.

19. Verbindung nach einem der Ansprüche 1, 17 oder 18, dadurch gekennzeichnet, daß das Pyranosid ein L-Fucosid ist.

20. Verfahren zur Herstellung einer Verbindung der Formel I gemäß einem Ansprüche 1 bis 19, dadurch gekennzeichnet, daß man zunächst unter Alkylierung, Acylierung oder Glycosylierung einer funktionellen Gruppe L¹ oder zweier funktioneller Gruppen L¹ und L², gleichzeitig oder nacheinander, eines Akzeptors der Formel II 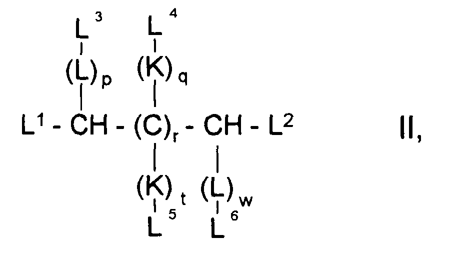 bei welchem die übrigen funktionellen Gruppen sowie die funktionellen Gruppen Lⁿ (n = 2-6 bzw. 3-6) notwendigenfalls geschützt vorliegen, mittels eines mit einer aktivierten funktionellen Gruppe L⁷ versehenen Donors III,
L⁸ - A - (Y)ₙ - B - L⁷ III
und für den Fall, daß auch L² ungeschützt vorliegt, mittels einer mit einer aktivierten funktionellen Gruppe L⁷ versehenen Donors IV
L⁷ - (D)ₐ - (Q)ᵣ - E - L⁸ IV,
deren übrige funktionelle Gruppe und die funktionelle Gruppe L⁸ notwendigenfalls Schutzrgruppen tragen, Zwischenverbindung V 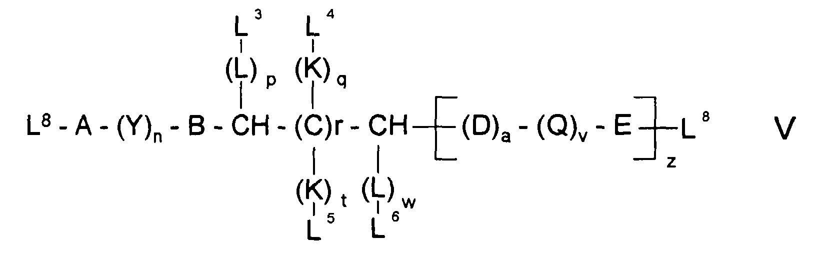 herstellt, wobei die Donoren III und IV verschieden oder identisch sein können, wonach die funktionellen Gruppen der Zwischenverbindung V an den Atomgruppen K und L und gegebenenfalls die Schutzgruppen der funktionellen Gruppen L³ bis L⁶ und L⁸ selektiv entfernt werden und die selektiv entschützte Zwischenverbindung V mit einem oder mehreren Glycosyl- oder Polyoldonoren VI
L⁹ - Z VI
umgesetzt wird, wobei L⁹ eine aktivierte funktionelle Gruppe darstellt und die übrigen funktionellen Gruppe des Donors VI notwendigenfalls geschützt sind, und nach selektiver Entschützung der funktionellen Gruppen L⁸ mit dem Alkyldonor VII 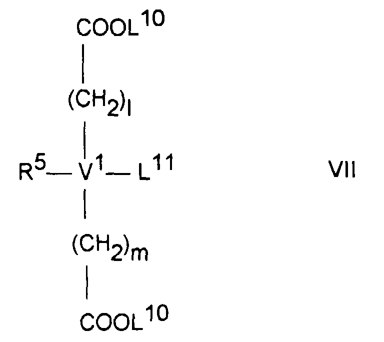 und gegebenenfalls mit dem
Alkyldonor VIII 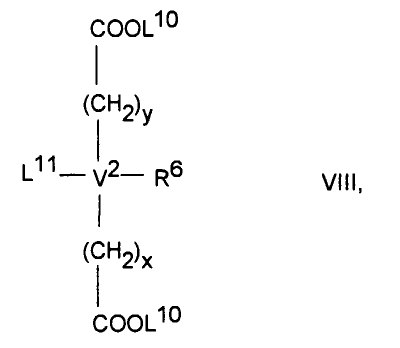 wobei L¹⁰ eine Carboxylschutzgruppe und L¹¹, gegebenenfalls in Verbindung mit V¹ bzw. V², eine alkylierende Gruppe darstellt, alkyliert und schließlich durch Entfernung sämtlicher Schutzgruppen in eine Verbindung der Formel I gemäß einem der Ansprüche 1 bis 19 überführt wird, wobei sämtliche nicht definierten Variablen die in Anspruch 1 genannten Bedeutungen haben.

21. Verfahren nach Anspruch 20, dadurch gekennzeichnet, daß man Akzeptor II zunächst mit einem oder mehreren Glycosyl- oder Polyoldonoren der Formel VI und anschließend mit Donor III und gegebenenfalls mit Donor IV umsetzt.

22. Arzneimittel enthaltend mindestens eine Verbindung der Formel I gemäß einem der Ansprüche 1 bis 19 oder deren pharmakologisch verträgliche Salze.

23. Verwendung der Verbindung der Formel I gemäß einem der Ansprüche 1 bis 19 zur Herstellung eines Arzneimittels für die Therapie oder Prophylaxe einer Krankheit, die mit einer übermäßigen, durch Selektinrezeptoren vermittelten Zelladhäsion in dem von der Krankheit betroffenen Geweben einhergeht.

24. Verwendung nach Anspruch 23, dadurch gekennzeichnet, daß die Krankheit eine Autoimmunerkrankung ist.

25. Verwendung nach Anspruch 23, dadurch gekennzeichnet, daß die Krankheit eine Herz-Kreislauf-Erkrankung ist.

26. Verwendung der Verbindung der Formel I gemäß einem der Ansprüche 1 bis 19 für die Herstellung eines Mittels zur Diagnose einer Krankheit, welche mit einer übermäßigen, durch Selektinrezeptoren vermittelten Zelladhäsion in dem von der Krankheit betroffenen Gewebe einhergeht.
